# EUROPÄISCHE PATENTSCHRIFT

(11) **EP 1 706 033 B1**
(45) Veröffentlichungstag und Bekanntmachung des Hinweises auf die Patenterteilung: **14.07.2010**
(21) Anmeldenummer: 05714892.6
(22) Anmeldetag: 20.01.2005
(51) Int. Cl.: A61B 5/15, A01K 67/033, A01K 1/06, A01K 1/02

(54) **VORRICHTUNG ZUR MINIMAL-INVASIVEN BLUTABNAHME BEI TIEREN MITTELS BLUTSAUGENDER RAUBWANZEN**
DEVICE FOR CARRYING OUT THE MINIMALLY INVASIVE WITHDRAWAL OF BLOOD FROM ANIMALS BY USING BLOOD-SUCKING ASSASSIN BUGS
DISPOSITIF DE PRELEVEMENT SANGUIN A INVASION MINIMALE POUR ANIMAUX PAR L'UTILISATION DE REDUVES HEMATOPHAGES

(30) Priorität: 20.01.2004 DE 102004004066
(43) Veröffentlichungstag der Anmeldung: 04.10.2006
(73) Patentinhaber: Forschungsverbund Berlin e.V., 12489 Berlin (DE)
(72) Erfinder: THOMSEN-HÄFLIGER, Ruth, 12587 Berlin (DE); VOIGT, Christian, 10245 Berlin (DE)
(74) Vertreter: Hengelhaupt, Jürgen
(86) Internationale Anmeldenummer: PCT/DE2005/000087
(87) Internationale Veröffentlichungsnummer: WO 2005/067798

(56) Entgegenhaltungen:
- US-A- 5 464 360
- US-A- 5 849 262

## Beschreibung

Die Erfindung betrifft eine Vorrichtung zur minimal-invasiven Blutabnahme an Tieren mittels blutsaugender Raubwanzen zu Forschungs-, Versuchs- und Diagnosezwecken.

Blutproben werden im Rahmen vieler Versuchsanordnungen benötigt, weshalb Blutabnahmen zu den sehr häufigen Eingriffen zählen. Zum einen werden für die Zulassungen von Medikamenten Blutabnahmen in Tierversuchen benötigt, die die Unbedenklichkeit der Inhaltsstoffe für die spätere menschliche Anwendung bestätigen sollen. Zum anderen werden Blutabnahmen auch bei gewöhnlichen tierärztlichen Untersuchungen benötigt, um die Ursachen für die Erkrankungen eines Tieres herauszufinden.

Während Blutabnahmen bei großen Tieren infolge ihrer gut zugänglichen Gefäße und ihres großen Blutvolumens unproblematisch sind, stellen Blutabnahmen bei kleineren Tieren wegen der geringeren Blutmengen und der filigranen und oftmals schlecht zugänglichen Blutgefäße eine Schwierigkeit dar. Aufgrund dieser schlechten Blutabnahmemöglichkeiten wird bei Tierversuchen im Labormaßstab das gesetzliche Maß der im Versuch zulässigen Belastungen sehr weit ausgeschöpft. Neben der Tatsache, dass durch die Entnahme der Tod der Versuchstiere billigend in Kauf genommen und zum Teil gezielt herbeigeführt werden muss (terminale Blutabnahme aus der Herzkammer), stellt sich das weitergehende Problem, dass Diagnose und Kinetikstudien einer kontinuierlichen Blutabnahme bedürfen, um eine zeitliche Wirkstoffveränderung oder Konzentrationsabnahme von Medikamentenverabreichungen bestimmen zu können.

Bei Haustieren gestaltet sich eine Blutabnahme schwierig, da durch die Atmosphäre in einer Tierarztpraxis, eine Unruhe bzw. eine Gegenwehr des Tieres zu befürchten ist, die nur durch Zwang oder durch Anästhetika zu überwinden ist. Beides hat aber Einfluss auf das Blutbild des Tieres, so dass mit der gängigen Blutabnahme ein Ausschluss externer Einflüsse auf das Blutbild nicht gewährleistet werden kann. Im Allgemeinen erfolgen Blutabnahmen durch besondere Instrumente wie eine Spritze oder Kappilarröhrchen. Dabei wird durch Punktion einer von außen zugänglichen Vene eine Blutabnahme durchgeführt. Je nach Größe der Fokustiere ist jedoch das Volumen der abzunehmenden Blutmenge begrenzt und die Zugänglichkeit der Gefäße schwierig. Die Größe der Entnahmevolumina muss unter Berücksichtigung der Entnahmefrequenz, den Grenzen der pathophysiologischen Kompensationsmechanismen und der Regenerationszeit entsprechend gewählt werden. Die bei einem Tier entnehmbare Blutmenge hängt jedoch nicht nur vom maximal entnehmbaren Blutvolumen ab (sie beträgt z.B. bei einer Ratte mit einem Körpergewicht von 100g und 5ml Gesamtblutvolumen 1ml. Das entspricht 20%), sondern auch von der Technik der Blutabnahme und dem Geschick der ausführenden Person. Daher müssen Abnahmetechniken gewählt werden, die das entsprechende Tier nicht übermäßig stark belasten und eine konstante und ausreichende Blutmenge gewährleisten. Bei der Blutabnahme an kleinen Tieren (mit weniger als ca. 3 kg Körpergewicht) können so nur Blutmengen von weniger als 1 ml entnommen werden, die bei den gebräuchlichen Abnahmetechniken z. B. der Punktion des Herzens, das An- oder oftmals Durchstechen der Venen oder ein wiederholtes, stückweises Abtrennen des Schwanzes erfordern.

Auch das Anstechen der Zungenvenen von Ratten ist als eine Blutabnahmemöglichkeit beschrieben worden [Bundesamt für Veterinärwesen: "Blutabnahme bei Labornagetieren und Kaninchen zu Versuchszwecken", Information Tierschutz 3.02, Seite 7], bei der die Zunge des Versuchstieres mit den Fingern ergriffen wird und mit einer dünnen Nadel angestochen wird. Diese Prozedur kann zwar mehrmals wiederholt werden. Allerdings ist das Tier bei einer anschließenden Futterverweigerung von mehr als einem Tag zu töten, womit ein reproduzierbares und aussagekräftiges Versuchsergebnis oftmals nicht abschließend gewährleistet werden kann. Die Punktion wird zudem unter Anästhesie durchgeführt, was zu einer Veränderung der Blutzusammensetzung führt und als ein weiterer Punkt der Reproduzierbarkeit des Versuchsansatzes entgegensteht.

Eine weitere Blutabnahmetechnik stellt die retrobulbäre Blutabnahme dar, bei der der retrobulbäre Venenplexus hinter dem Auge punktiert wird. Die anatomischen Nähe zum Auge stellt neben der praktischen Schwierigkeit der exakten Lokalisierung der Augenvene auch ein normatives Problem dar, da bei wiederholter Entnahme Fehlmanipulationen wie Hämatome am Auge oder Blindheit auftreten können. Gem. §§ 13 I, 13a II TSchG sind belastende Tierversuche auf das unerlässliche Maß zu beschränken bzw. nur mit einer Bewilligung durchzuführen. Dabei ist, wie in der Beschreibung der: Tierärztliche Vereinigung für Tierschutz e.V., "Hinweise zur Blutabnahme bei kleinen Versuchstieren, Seite 3 dargelegt, der Einsatz dieser Abnahmetechnik mit einem Anästhetikum durchzuführen und die dadurch erhaltenen Blutmengen sind begrenzt, inkonstant und mit Fremdstoffen kontaminiert. Bei allen bekannten Methoden wird durch ein medizinisches Instrument eine Punktion einer Blutbahn durchgeführt und damit Blut entnommen. Die dabei anfallenden Volumina sind aber gering und eine exakte Lokalisation der Blutgefäße kann von außen nicht immer gewährleistet werden. Zudem müssen, wie bei den oben genannten Methoden beschrieben, die Tiere gegebenenfalls anästhesiert werden.

Eine völlig andere Methode der Blutabnahme bei kleinen Tieren wird von O. v. Helversen, M. Volleth und J. Núnez in "A new method for obtaining blood from a small mammal without injuring the animal: use of Triatomid bugs", Experientia 42 (1986) S. 809 und 810 beschrieben. Zur Abnahme geringer Blutmengen von kleinen Säugetieren wurden Raubwanzen verwendet, die beispielsweise von Fledermäusen Blut gesaugt haben. Das im Abdomen der Raubwanzen gespeicherte Blut der Fledermäuse wurde mittels üblicher Kanülen aus der Wanze abgenommen und konnte für diverse Untersuchungen verwendet werden. Oftmals gestaltete es sich jedoch als schwierig, die Wanze nach der Blutmahlzeit zu fixieren, da diese schnell an dunkle Orte zu flüchten versuchen.

Von C.C.Voigt, M. Faßbender, M. Dehnhard, G. Wibbelt, K. Jegenow wird in "Validation of a minimally invasive blood-sampling technique for the analysis of hormones in domestic rabbits, Oryctolagus cuniculus (Lagomorpha)" in General and Comparative Endocrinology 135 (2004) S. 100-107, verglichen wie sich die Blutzusammensetzung anhand hormoneller Konzentrationsveränderungen im Blut bei der Durchführung von Blutentnahmen mit Kanülen im Gegensatz zu Blutentnahmen mit Raubwanzen verändert. Es konnte in dieser Versuchsreihe anhand von Kortisolmessungen festgestellt werden, dass der Streßfaktor der Versuchstiere bei der Durchführung der Blutentnahmetechnik mit Kanülen größer ist als bei der Blutentnahme durch Raubwanzen. Darüberhinaus verändert die Blutentnahme durch Raubwanzen die Blutzusammensetzung nicht und stellt ein gegenüber der Blutentnahmetechnik mit Kanülen stressfreieres Verfahren zur Blutentnahme bei Tieren dar.

Aufgabe der Erfindung ist es, die negativen Wirkungen der üblichen konventionellen Blutabnahmetechniken zu überwinden. Es soll zum einen gewährleistet werden, dass bei kleinen Tieren, die bislang mit konventionellen Blutabnahmemethoden zu Schaden kommen, eine schonende Blutabnahme erfolgen kann, die zusätzlich wiederholt angewandt werden kann und die die Ergebnisse nicht verfälscht. Zum anderen sollen Tierarten, bei denen es bislang überhaupt nicht möglich ist, Blut abzunehmen, mit der Raubwanze beprobt werden können. Des weiteren soll die Wanze nach der Blutmahlzeit einfach fixiert werden können, um ihr Fliehen zu vermeiden und so das Blut problemlos aus ihrem Abdomen saugen zu können.

Aus US-A-5464360 ist ein Behälter mit perforiertem außenwand und bewegbaren Schieber bekannt.

Die Aufgabe wird erfindungsgemäß durch eine Vorrichtung mit den Merkmalen des Anspruchs 1 gelöst, indem diese aus mindestens einem Behälter besteht, der mindestens eine perforierte Außenwand aufweist, in dem die Raubwanze derart am Tier positionierbar ist, dass die Raubwanze von diesem Tier Blut saugen kann. Anschließend lässt sich die Raubwanze in diesem Behälter mittels einer bewegbaren Zwischenwand derart an einer Stelle der perforierten Außenwand fixieren, dass das aus dem Lebewesen gesaugte und im Abdomen gespeicherte Blut der Raubwanze mittels einer Kanüle direkt und schnell aus der Wanze entnommen werden kann.

In einer Ausgestaltung besteht der Behälter aus einem Hohlzylinder, der an seiner einen Bodenfläche perforiert ist, mit der die in diesem Behälter befindliche Raubwanze an dem Lebewesen positioniert werden kann. Nachdem die Raubwanze durch diese perforierte Bodenfläche aus dem Tier Blut gesaugt hat, kann die Raubwanze mittels einer an der gegenüberliegenden Bodenfläche gehalterten Kolbenstange und dem daran befestigten Kolben, der die bewegbare Zwischenwand darstellt, sanft an einer Stelle der perforierten Bodenfläche fixiert werden, so dass die Blutabnahme aus dem Abdomen der Wanze erfolgen kann.

In einer Ausgestaltung der Erfindung besteht die Vorrichtung aus zwei benachbarten Behältern, wobei sich die Raubwanze in dem ersten Behälter befindet und das zu beprobende Tier in dem zweiten Behälter. Zwischen diesen beiden Behältern ist eine zumindest teilweise perforierte Trennwand angeordnet. Das Tier, wird mittels eines beweglichen Schiebers derart an der perforierten Trennwand fixiert, dass die Raubwanze saugen kann. Der erste Behälter, in dem sich die Raubwanze befindet, verfügt, analog zu der ersten bevorzugten Ausführungsform, über eine bewegbare Zwischenwand mit der die Raubwanze mittels der beweglichen Kolbenstange an der perforierten Bodenfläche fixiert werden kann.

Eine Ausgestaltung besteht darin, einen annähernd kugelförmigen Behälter mit mindestens einer perforierten Außenwand und einer elastischen und verformbaren Zwischenwand zu versehen, wobei die Zwischenwand in Abhängigkeit von der Materialeigenschaft ihre Lage und Form durch von außen einwirkende Kräfte verändern kann und dazu geeignet ist, die Wanze zwischen perforierter Außenwand und Zwischenwand zu fixieren. Die Form- und Lageveränderung der Zwischenwand kann beispielweise durch mechanische Einwirkung erfolgen. Nach Ende des Saugvorganges kann so die Wanze zur Blutabnahme in dem kugelförmigen Behälter fixiert werden.

Als denkbare Möglichkeit könnte ein verformbarer Behälter mit maschenartiger Gewebestruktur dienen. Die Raubwanze kann zur Blutabnahme in dem flexiblen Behälter mittels einer flexiblen Haltevorrichtung am Fokustier so positioniert werden, dass sie Blut saugen und nach Ende des Saugvorganges mittels der flexiblen Haltevorrichtung vom Fokustier wieder entfernt werden kann. Durch Zusammenfalten der maschenartigen Gewebestruktur kann die Wanze dann im Behälter fixiert werden. Die Blutabnahme der Wanze erfolgt analog der vorstehenden Ausgestaltungsvarianten mit einer Kanüle.

Die Vorrichtung zur Blutabnahme durch Raubwanzen hat gegenüber dem Stand der Technik den Vorteil, dass die zur selbständigen Blutabnahme fähige Raubwanze in einem abgeschlossenen Behälter untergebracht ist, die durch eine perforierte Zwischenwand an bestimmte Körperbereiche bzw. - zonen des zu untersuchenden Tieres zur Blutabnahme gelangen kann und anschließend zur Entnahme des Blutes fixierbar ist. Hat die Wanze eine Vene gefunden, so sticht sie das Fokustier und beginnt mit dem Saugvorgang. Die Raubwanze erkennt bei entsprechender Nähe (ca. 50cm) das Fokustier durch dessen Körpertemperatur und sticht zu, sobald sie die Möglichkeit dazu hat. Der komplette Saugvorgang dauert in der Regel zwischen vier bis acht Minuten und kann je nach benötigter Blutmenge auch öfter, d.h. von immer neuen Wanzen, wiederholt werden. Der Biss und das Saugen wird von dem Tier nicht bemerkt, so dass von einer Beeinträchtigung des Tieres durch die Applikation der Wanze nicht auszugehen ist. Es bleibt keine Wunde zurück. Zudem wird das Blut des Versuchstieres durch die Speicherung im Abdomen weitgehend nicht verändert, wie es in Experimentia 42 (1986): "A new method for obtaining blood from a small mammal without injuring the animal: use of triatomid bugs" von O.v.Helversen, M.Volleth und J. Núnez beschrieben ist. Auch ist eine mehrfache Blutabnahme mit differenziertem definiertem Volumen, bedingt durch Verwendung unterschiedlicher Entwicklungsstadien (i.e. Größen) von Raubwanzen, möglich. Die Abnahmemenge beträgt bis zu 4ml je nach Größe und Entwicklungsstadium der Wanze. Das Blut steht sofort nach der Entnahme aus der Wanze für Untersuchungen zur Verfügung. Das Blut hat durch die Aufnahme und Speicherung im Abdomen der Wanze seine Zusammensetzung und seine molekularen Eigenschaften weitgehend nicht verändert. Dieser Umstand ist in: "Validation of Non-Invasive Blood-Sampling Technique for Double-Labelled Water Experiments" von C. Voigt, O. v. Helversen, R. Michener und T. Kunz in Journal of Experimental Zoology 296A: 87-97 [88] (2003) beschrieben. Dort ist eine nahezu vollständige Kontaminationsfreiheit des entnommenen Blutes durch wanzeneigene Stoffe nachgewiesen worden. Es ist daher eine rückstands- und fremdstofffreie Untersuchung des gewonnenen Blutes für viele Teilbereiche in der Tiermedizin, der Wissenschaft und der pharmakologischen Forschung möglich.

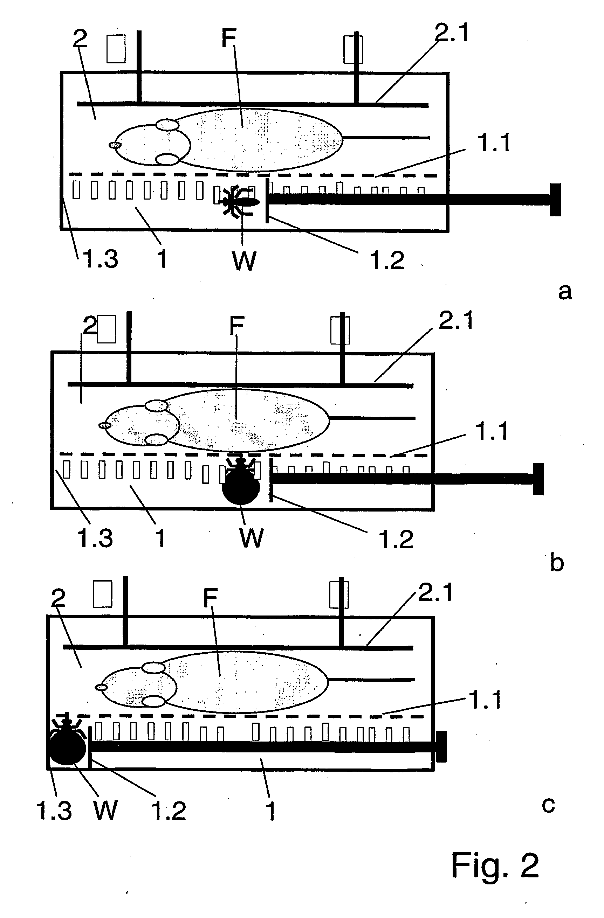

Die Erfindung wird nachstehend anhand von Ausführungsbeispiel gemäß Figur 2 näher erläutert; die weitere ausführungen sind kein Teil der Erfindung.

Die zugehörigen Zeichnungen stellen dar:
Fig. 1: Ausführung der Vorrichtung als Hohlzylinder
Fig. 2: Ausführung der Vorrichtung mit zwei benachbarten Behältern
Fig. 3: Ausführung der Vorrichtung mit einem kugelförmigen Behälter

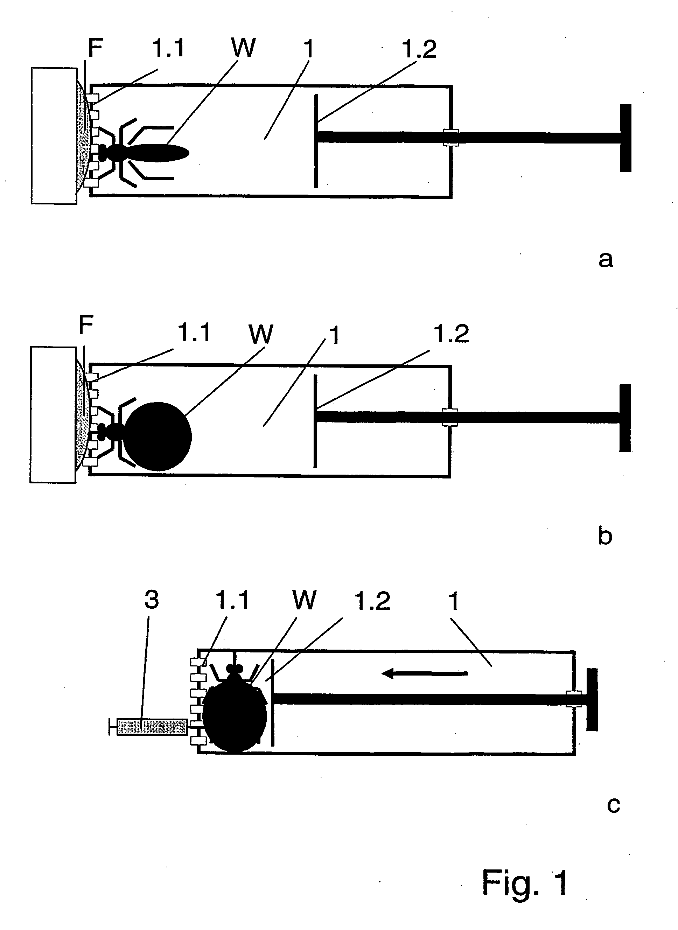

Bei einer Ausführung wie sie in Fig. **1** dargestellt ist, besteht der Behälter **1** der Vorrichtung aus einem Hohlzylinder dessen eine Bodenfläche **1.1** perforiert ist. An der gegenüberliegenden Bodenfläche ist eine Kolbenstange mit einem daran befestigten Kolben gehaltert, der die bewegbare Zwischenwand **1.2** darstellt.

Wie in Fig. 1a gezeigt, befindet sich in diesem Behälter **1,** zwischen der perforierten Bodenfläche **1.1** und der bewegbaren Zwischenwand **1.2,** die Raubwanze **W.** Der Behälter **1** wird nun mit seiner perforierten Bodenfläche **1.1** derart am Fokustier **F** positioniert, dass die Wanze zur Blutabnahme an das Fokustier F herankommt und mit ihrem Saugrüssel durch die perforierte Bodenfläche stechen und **1.1** das Blut aus dem Fokustier **F** saugen kann. Die Fig. 1b zeigt die Wanze **W** in dem Behälter **1** an der perforierten Außenwand **1.1** kurz vor Ende des Saugvorganges, erkennbar an dem angeschwollenen Abdomen der Wanze **W.**

In Fig. **1c** ist zu erkennen, dass die Wanze nach Ende des Saugvorganges mittels einer bewegbaren Zwischenwand **1.2,** die über eine an der gegenüberliegenden Bodenfläche gehalterte Kolbenstange an die perforierte Bodenfläche **1.1** niedergedrückt ist, wodurch die Bewegungsfreiheit der Wanze auf nahezu Null eingeschränkt worden ist, fixiert ist. Mit Spritze und Kanüle **3** kann dann von der fixierten Wanze **W** das Blut aus dem angeschwollenen Abdomen abgezogen werden.

Eine Ausführung der erfindungsgemäßen Vorrichtung ist in der Fig. **2** dargestellt. Hierbei besteht die erfindungsgemäße Vorrichtung aus zwei benachbarten Behältern **1, 2,** wobei die zumindest teilweise perforierte Außenwand des Behälters **1** die gemeinsame Trennwand **1.1** zum zweiten Behälter **2** darstellt. Dabei befindet sich die Raubwanze **W** in dem ersten Behälter **1** und das Fokustier F in dem zweiten Behälter **2.**

In der Fig. 2a ist dargestellt, dass das Fokustier **F,** in diesem Fall ein Kleinsäuger von dem das Blut abgenommen werden soll, mittels eines beweglichen Schiebers **2.1** derart an der perforierten Trennwand **1.1** fixiert wird, dass die Raubwanze W zustechen und Blut saugen kann.

Die Fig. 2b zeigt wiederum die Wanze **W** in dem ersten Behälter **1** an der perforierten Trennwand **1.1** kurz vor Ende der Blutaufnahme, was an dem angeschwollenen Abdomen der Wanze **W** erkennbar ist.

Die Fig. 2c zeigt, wie nach beendeter Blutaufnahme durch die Raubwanze **W** diese, analog zu der ersten bevorzugten Ausführungsform gem. Fig. 1, mittels der bewegbaren Zwischenwand **1.2** an einer zweiten perforierten Außenwand **1.3** fixiert wird. Aus der so fixierten Raubwanze **W** kann nun mit Spritze und Kanüle **3** das aus dem Fokustier **F** abgesaugte Blut wieder aus der Wanze **W** entnommen werden.

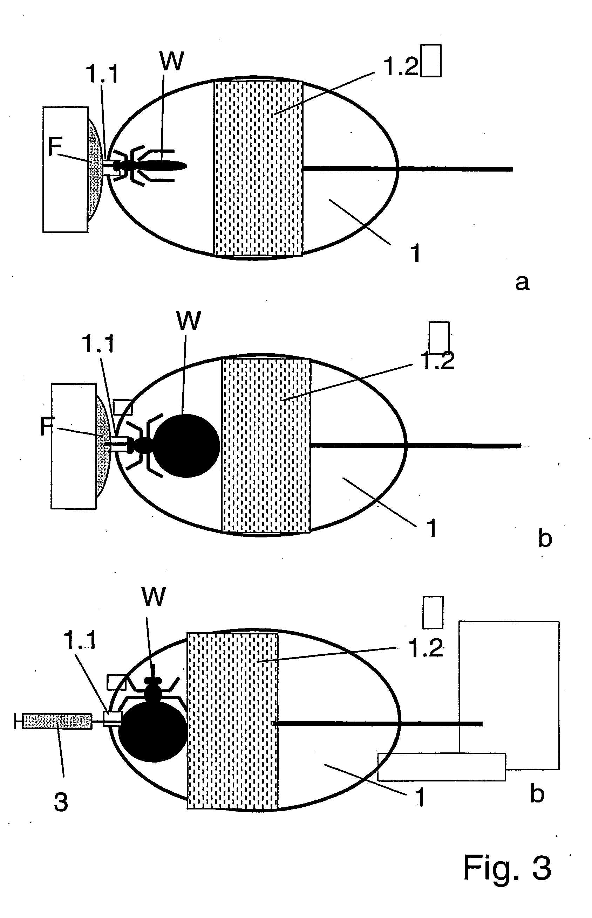

Wie in Fig. 3 dargestellt, besteht eine Vorrichtung darin, einen annähernd kugelförmigen Behälter **1** mit einer teilweise perforierten Außenwand **1.1** und einer bewegbaren Zwischenwand **1.2** zu versehen, die elastisch verformbar ist. Die Zwischenwand **1.2** ist beispielsweise ein Schaumgummikörper, der etwa in der Mitte des Behälters **1** am gesamten Umfang an der Außenwand des kugelförmigen Behälters anliegt. Die Raubwanze **W** befindet sich in dem einen Teil des kugelförmigen Behälters **1** zwischen der Zwischenwand **1.2** und der perforierten Außenwand **1.1.** Nach Ende des Saugvorganges kann die Wanze **W** zur Blutabnahme mit einer Spritze und Kanüle **3** in dem kugelförmigen Behälter **1** zwischen der perforierten Außenwand **1.1** und der bewegbaren Zwischenwand **1.2** fixiert werden. Das geschieht beispielsweise dadurch, dass durch mechanisches Verschieben der verformbaren Zwischenwand **1.2** zuerst die flexible Zwischenwand **1.2** an der Wanze **W** anliegt und durch weiteres Verschieben der verformbaren Zwischenwand **1.2** die Wanze **W** an der perforierten Außenwand **1.1** fixiert wird. Dieser kugel- oder eiförmige Behälter ist vorzugsweise als Vorrichtung zur Blutabnahme bei Vögeln geeignet, indem dieser Behälter in die entsprechenden Nester gelegt wird.

Weiterhin kann ein flexibel verformbarer Behälter **1** mit maschenartiger Gewebestruktur als eine Ausführungsvariante der Vorrichtung dienen, die mittels einer flexiblen Haltevorrichtung, die beispielsweise aus einer Angelrute sowie einer daran befestigten Angelschnur besteht, derart am Fokustier **F** positioniert werden, dass die Wanze dort das Blut vom Fokustier **F** saugen kann. Nach Ende des Saugvorganges wird der flexibel verformbare Behälter **1** mit der darin befindlichen Raubwanze **W** mittels der Angelrute und Angelschnur vom Fokustier **F** entfernt. Ein zur Blutabnahme erforderliches Fixieren der Wanze **W** kann beispielsweise durch ein Zusammenfalten der maschenartigen Gewebestruktur des Behälters **1** erreicht werden. Die Blutabnahme aus der Raubwanze **W** erfolgt analog der vorstehenden Ausführungsvarianten mittels Kanüle und Spritze **3.**

## Patentansprüche

1. Vorrichtung zur minimal-invasiven Blutabnahme bei Tieren mittels blutsaugender Raubwanzen
**dadurch gekennzeichnet, dass**
die Vorrichtung aus einem ersten Behälter (1), der eine erste perforierte Außenwand (1.1), eine zweite perforierte Außenwand (1.3) und eine bewegbare Zwischenwand (1.2) aufweist, und einem zweiten Behälter (2), der mit dem ersten Behälter (1) verbunden ist, besteht, wobei die erste perforierte Außenwand (1.1) des ersten Behälters (1) die gemeinsame Trennwand (1.1) zu dem zweiten Behälter (2) darstellt, wobei sich die Raubwanze (W) in dem ersten Behälter (1) befinden kann und ein Fokustier (F) in dem zweiten Behälter (2) an der perforierten gemeinsamen Trennwand (1.1) positionierbar ist, so dass eine Blutaufnahme durch die Raubwanze (W) an dem Fokustier (F) erfolgen kann und nach erfolgter Blutaufnahme, die Raubwanze (W) mittels der bewegbaren Zwischenwand (1.2) an der zweiten perforierten Außenwand (1.3) fixierbar ist, derart, dass das Blut aus dem Abdomen der Raubwanze (W) mittels Injektionsspritze wieder entnehmbar ist.

2. Vorrichtung nach Anspruch 1
**dadurch gekennzeichnet, dass**
der zweite Behälter (2) einen beweglichen Schieber (2.1) aufweist, mit welchem das Fokustier (F) in dem zweiten Behälter (2) an der perforierten gemeinsamen Trennwand (1.1) positionierbar ist.

## Claims

1. Device for carrying out the minimally invasive withdrawal of blood from animals by using blood-sucking assassin bugs
**characterized in that**
the device consists of a first container (1), which comprises a first perforated outer wall (1.1), a second perforated outer wall (1.3) and a movable intermediate wall (1.2), and a second container (2) which is connected to the first container (1), wherein the first perforated outer wall (1.1) of the first container (1) is the shared separating wall (1.1) to the second container (2), wherein the assassin bug (W) can be located in the first container (1), and a focus animal (F) can be positioned in the second container (2) on the perforated, shared separating wall (1.1), so that blood can be removed from the focus animal (F) by the assassin bug (W), and after blood has been removed, the assassin bug (W) can be affixed by means of the movable intermediate wall (1.2) to the second perforated outer wall (1.3) in such a manner that the blood can again be removed from the abdomen of the assassin bug (W) by means of an syringe.

2. A device according to claim 1
**characterized in that**
the second container (2) comprises a movable slide (2.1), with which the focus animal (F) can be positioned in the second container (2) on the perforated shared separating wall (1.1).

## Revendications

1. Dispositif de prélèvement sanguin à invasion minimale pour animaux par l'utilisation de réduves hématophages
**caractérisé en ce que**
le dispositif se compose d'un premier conteneur (1) qui présente une première paroi extérieure (1.1) perforée, une deuxième paroi extérieure (1.3) perforée et une paroi intermédiaire (1.2) mobile, et d'un deuxième conteneur (2) qui est raccordé au premier conteneur (1), la première paroi extérieure (1.1) perforée du premier conteneur (1) représentant la paroi de séparation (1.1) commune par rapport au deuxième conteneur (2), le réduve hématophage (W) pouvant se trouver dans le premier conteneur (1), et un animal cible (F) pouvant être positionné dans le deuxième conteneur (2) au niveau de la paroi de séparation (1.1) commune perforée de sorte qu'un prélèvement sanguin par le réduve hématophage (W) sur l'animal cible (F) peut être effectué et de sorte que, une fois le prélèvement sanguin effectué, le réduve hématophage (W) peut, au moyen de la paroi intermédiaire (1.2) mobile, être fixé sur la deuxième paroi extérieure (1.3) perforée de sorte que le sang peut être extrait à son tour de l'abdomen du réduve hématophage (W) au moyen d'une aiguille hypodermique.

2. Dispositif selon la revendication 1,
**caractérisé en ce que**
le deuxième conteneur (2) présente un curseur (2.1) mobile avec lequel l'animal cible (F) dans le deuxième conteneur (2) peut être positionné sur la paroi de séparation (1.1) commune perforée.
